# EUROPEAN PATENT SPECIFICATION

(11) **EP 4 361 591 B1**
(45) Date of publication and mention of the grant of the patent: **06.08.2025**
(21) Application number: 23168000.0
(22) Date of filing: 14.04.2023
(51) Int. Cl.: G01N 1/30, G01N 15/14, G01N 1/31, B01L 3/02, B01L 3/00, G01N 15/00, G01N 15/10

(54) **BIOLOGICAL PARTICLE ANALYSIS METHOD**
VERFAHREN ZUR ANALYSE BIOLOGISCHER PARTIKEL
PROCÉDÉ D'ANALYSE DE PARTICULES BIOLOGIQUES

(30) Priority: 27.10.2022 US 202263419834 P
(43) Date of publication of application: 01.05.2024
(73) Proprietor: Cytoaurora Biotechnologies, Inc., Zhubei City, Hsinchu County 30261 (TW)
(72) Inventor: HUANG, Chung-Er, 30261 Zhubei City, Hsinchu County (TW); CHEN, Sheng-Wen, 30261 Zhubei City, Hsinchu County (TW); HO, Hsin-Cheng, 30261 Zhubei City, Hsinchu County (TW); YE, Guang-Ci, 30261 Zhubei City, Hsinchu County (TW)
(74) Representative: Viering, Jentschura & Partner mbB Patent- und Rechtsanwälte

(56) References cited:
- US-A1- 2016 129 443
- US-A1- 2021 293 778
- US-A1- 2021 382 062
- JOU HEI-JEN ET AL: "An Automatic Platform Based on Nanostructured Microfluidic Chip for Isolating and Identification of Circulating Tumor Cells", MICROMACHINES, vol. 12, no. 5, 21 April 2021 (2021-04-21), pages 473, XP093083914, DOI: 10.3390/mi12050473

## Description

### FIELD OF THE DISCLOSURE

The present disclosure relates to a biological particle analysis method, and more particularly to a biological particle analysis method having a multi-fluorescence staining process.

### BACKGROUND OF THE DISCLOSURE

US 2021/382062 A1 discloses an analysis apparatus (1) includes a housing unit (10) that houses a biological sample (S), a staining dispenser unit (20) that fluorescently stains the biological sample (S), an image acquisition unit (30) that acquires an image of the biological sample (S), a washer unit (40) that destains the biological sample (S), a drive unit (50), and a control unit (90) that analyzes the image and controls the drive unit (50). The control unit (90) performs adjustments so that the staining dispenser unit (20) and the housing unit (10) satisfy a first position condition, performs adjustments so that the image acquisition unit (30) and the housing unit (10) satisfy a second position condition, and performs adjustments so that the washer unit (40) and the housing unit (10) satisfy a third position condition. The analysis apparatus (1) maximizes the actual photography range of information related to the biological sample. The analysis apparatus (1) can also minimize variation in the time required for processes and can achieve high-quality analysis.

US 2016/129443 A1 discloses a method and a system for the isolation of picoliter droplets from a continuous stream of carrier fluid in a microfluidic system. In particular the disclosure relates to a system that comprises a sorter, a detector and a collector. The sorter serves for a selection of picoliter droplets with a desired content based upon an optical signal obtained from the picoliter droplets. The selected picoliter droplets are guided towards the detector that comprises a detection channel that is arranged perpendicular to a light beam. By means of a photodetector that is aligned with the optical axis of the light beam the passage of the picoliter droplets can be detected. The collector comprises multiple wells and the detection of the passage of the picoliter droplets results in a relative movement of the detector and a collector such that the picoliter droplet is isolated by depositing it into one of said wells. The disclosure moreover relates to a method that uses such a system to isolate individual picoliter droplets. In particular the disclosure relates to a method that allows for an analysis and/or further processing of the content of the picoliter droplets that have been isolated from a microfluidic system by depositing them into a well.

US 2021/293778 A1 discloses a catcher, a capture device, and a method for capturing at least one target biological particle are provided. The catcher includes a base and a plurality of capture arms extending from the base and spaced apart from each other. Each of the capture arms has a free end portion configured to capture a target biological particle and a supporting segment connected between the free end portion and the base. The supporting segment of each of the capture arms is arranged in a projection space defined by orthogonally projecting the free end portion along a height direction onto the base. When the target biological particle is captured by two of the capture arms that are bent and arranged adjacent to each other, a part of the target biological particle is trapped by the supporting segments of the two of the capture arms and is held.

JOU HEI-JEN ET AL provides "An Automatic Platform Based on Nanostructured Microfluidic Chip for Isolating and Identification of Circulating Tumor Cells", MICROMACHINES, vol. 12, no. 5, 21 April 2021 (2021-04-21), page 473, XP093083914.

Since many separate processes are involved in a conventional analysis method for biological particles, from those relating to biological particles that are initially present in the liquid specimen to the subsequent expression of biological characteristics, it can be difficult to streamline all of these processes together. Specifically, different stages of the conventional analysis method can further involve various different processes, so that continuity between the processes is even more difficult to achieve.

### SUMMARY OF THE DISCLOSURE

In response to the above-referenced technical inadequacies, the present disclosure provides a biological particle analysis method to effectively improve on the issues associated with conventional analysis methods according to independent claim 1. The dependent claims show further embodiments of claim 1.

In order to solve the above-mentioned problems, one of the technical aspects adopted by the present disclosure is to provide a biological particle analysis method, which includes a staining step, an analyzing step, a capturing step, a washing step, and a characterization expressing step. The staining step is implemented by fluorescence staining a liquid specimen having a plurality of biological particles through a fluorescence staining process, so that at least one of the biological particles becomes a fluorescence color and is defined as at least one target biological particle. The analyzing step is implemented by accommodating the liquid specimen being fluorescence stained into a pico-droplet generator, and using a camera device to take a real-time image of the liquid specimen in the pico-droplet generator. Moreover, the pico-droplet generator includes a hollow needle for receiving the liquid specimen therein, a container and a top piezoelectric member disposed on the container, the container receives the liquid specimen, and the hollow needle is in fluid communication with the container by being connected to a bottom side of the container. Specifically, the top piezoelectric member is configured to vibrate the container for enabling the biological particles in the container to be arranged along a predetermined path of the hollow needle, the camera device corresponds in position to the hollow needle, and the camera device in the analyzing step is used to take the real-time image of the liquid specimen in a free end of the hollow needle. The capturing step is implemented by using the pico-droplet generator to output a target pico-droplet having the at least one target biological particle onto a biochip according to the real-time image. The at least one target biological particle in the target pico-droplet is captured by the biochip. The washing step is implemented by removing the fluorescent color of the at least one target biological particle in the target pico-droplet captured by the biochip through a washing process. The characterization expressing step is implemented by fluorescence staining the at least one target biological particle captured by the biochip for N number of times through the fluorescence staining process and the washing process, and using a recording device to obtain a plurality of fluorescence images respectively corresponding to N kinds of biological characterization expressions. Specifically, N is a positive integer within a range from 2 to 50 and each fluorescence staining of the at least one target biological particle is related to one of the N kinds of biological characterization expressions.

Therefore, the capturing step of the biological particle analysis method in the present disclosure can be implemented to achieve the enrichment effect by being cooperated with the staining step and the analyzing step. Moreover, the biological particle analysis method in the present disclosure can be implemented to effectively connect the capturing step, the washing step, and the characterization expressing step by using the biochip to capture the at least one target biological particle. Accordingly, the fluorescence images respectively corresponding to multiple kinds of biological characterization expressions can be obtained from the liquid specimen through the biological particle analysis method, thereby facilitating any evaluation and determination of the at least one target biological particle.

These and other aspects of the present disclosure will become apparent from the following description of the embodiment taken in conjunction with the following drawings and their captions, although variations and modifications therein may be affected without departing from the scope of the novel concepts of the disclosure.

### BRIEF DESCRIPTION OF THE DRAWINGS

The described embodiments may be better understood by reference to the following description and the accompanying drawings, in which:
FIG. 1 is a flowchart of a biological particle analysis method according to a first embodiment of the present disclosure;
FIG. 2 is a schematic perspective view of an identifying system according to the first embodiment of the present disclosure;
FIG. 3 is a schematic side view showing the identifying system operated to implement a staining step;
FIG. 4 is a schematic view showing a fluorescence staining process of FIG. 3;
FIG. 5 is a schematic side view showing the identifying system operated to implement an analyzing step;
FIG. 6 is a schematic cross-sectional view showing a part of FIG. 5;
FIG. 7 is a schematic view showing a camera device of the identifying system operated to take a real-time image of a pico-droplet generator;
FIG. 8 is a schematic side view showing the identifying system operated to implement a capturing step;
FIG. 9 is a schematic cross-sectional view showing a part of FIG. 8;
FIG. 10 is a schematic view showing a target biological particle captured by a biochip of the identifying system;
FIG. 11 is a schematic view showing the target biological particle captured by the biochip of the identifying system with another configuration;
FIG. 12 is a schematic view showing the pico-droplet generator of the identifying system outputting an abandoned pico-droplet;
FIG. 13 is a schematic side view showing the identifying system operated to implement a washing step;
FIG. 14 is a schematic side view showing the identifying system operated to implement a characterization expressing step;
FIG. 15 is a partial schematic cross-sectional view of the camera device and the pico-droplet generator of the identifying system; and
FIG. 16 is a schematic side view of the identifying system according to a second embodiment of the present disclosure.

### DETAILED DESCRIPTION OF THE EXEMPLARY EMBODIMENTS

The present disclosure is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present disclosure.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present disclosure or of any exemplified term. Likewise, the present disclosure is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

### [First Embodiment]

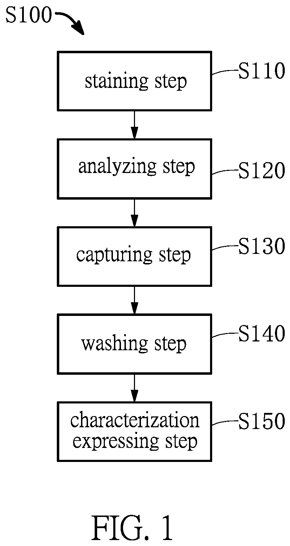

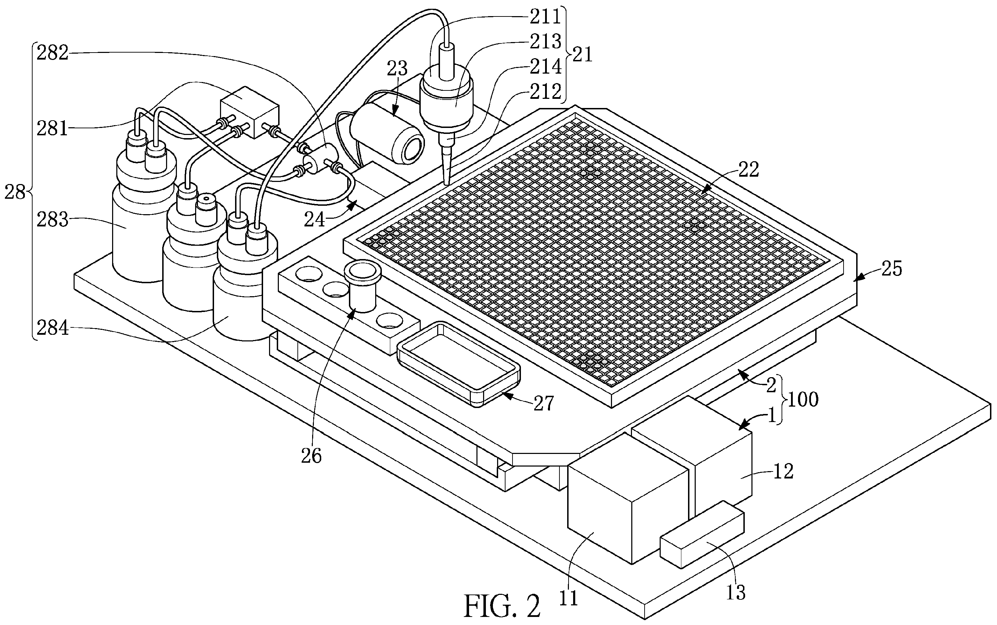

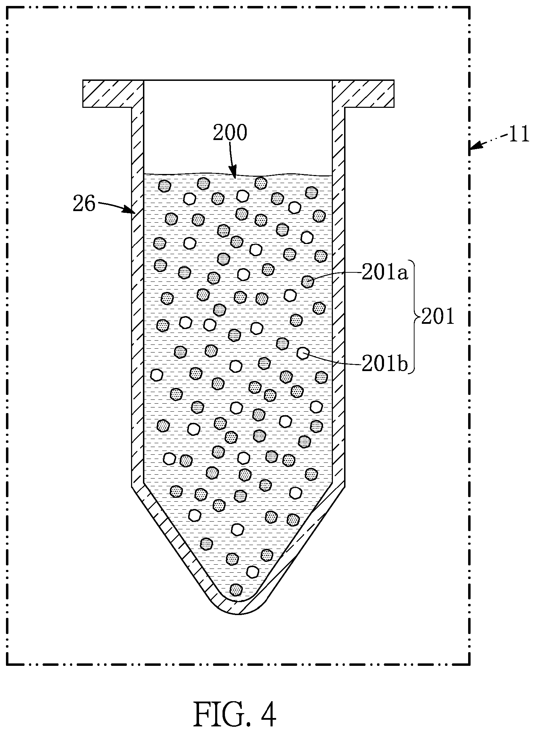

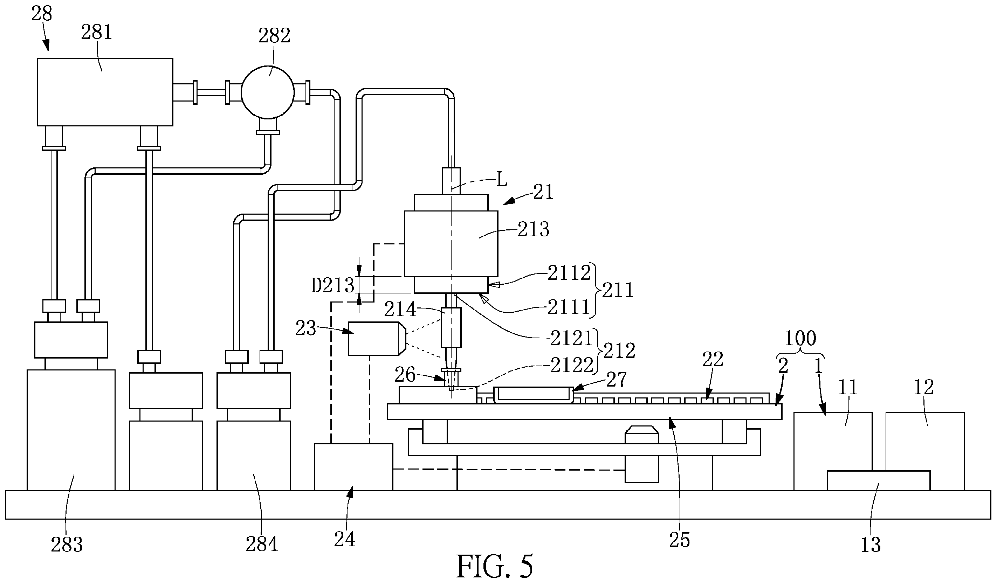

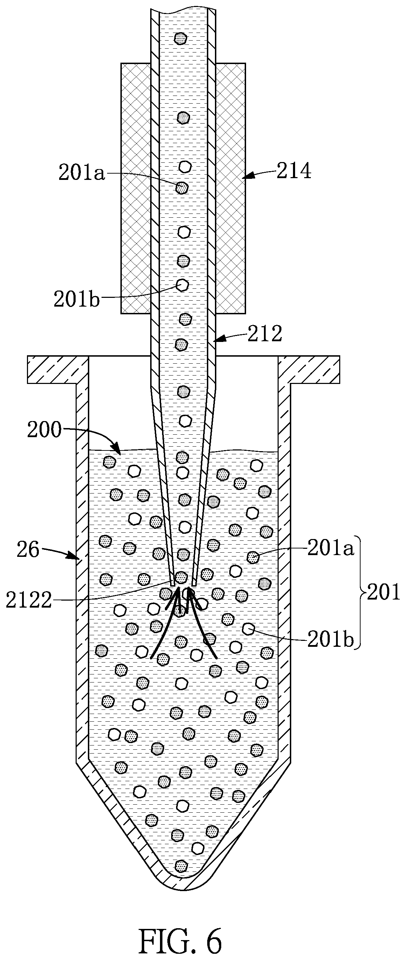

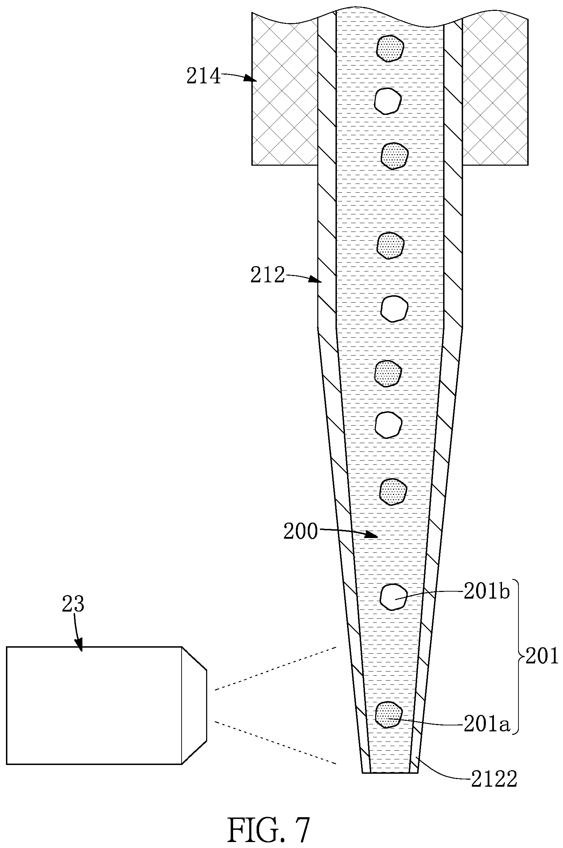

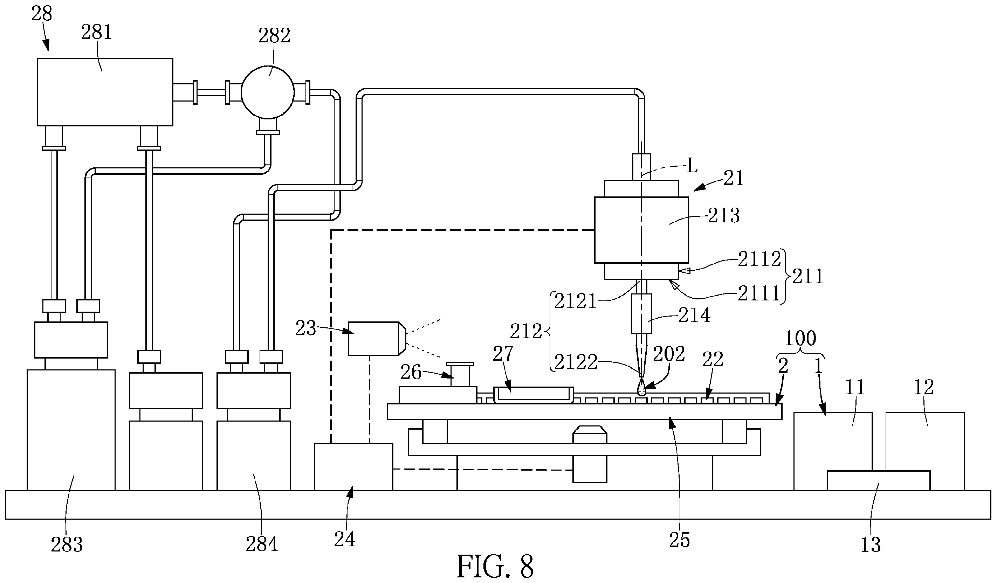

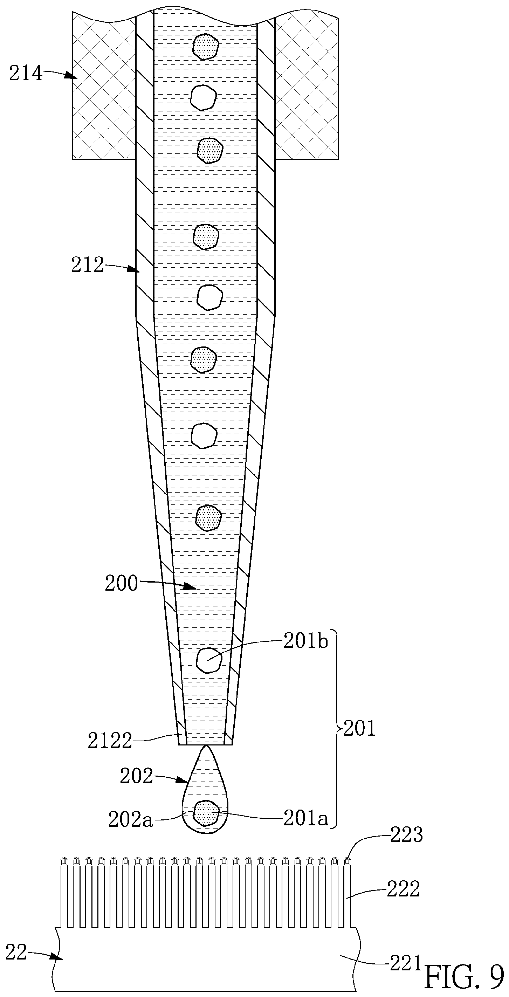

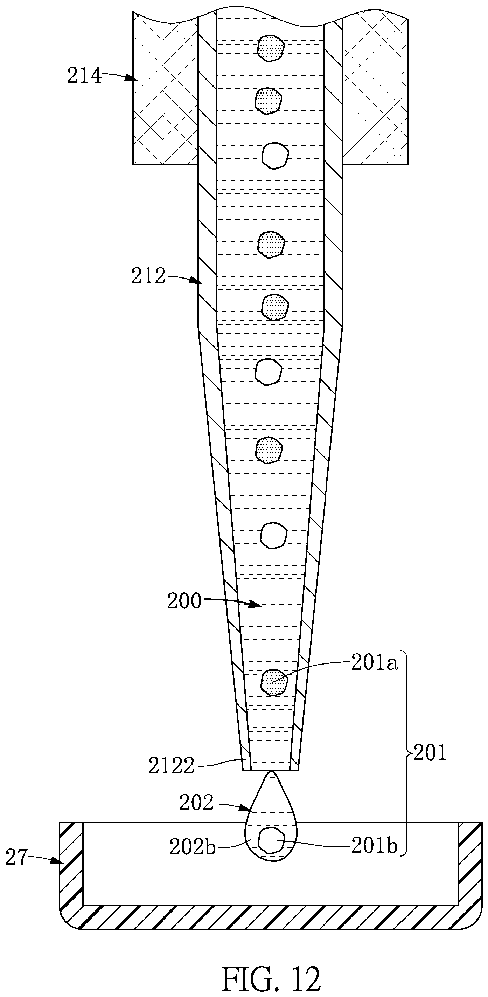

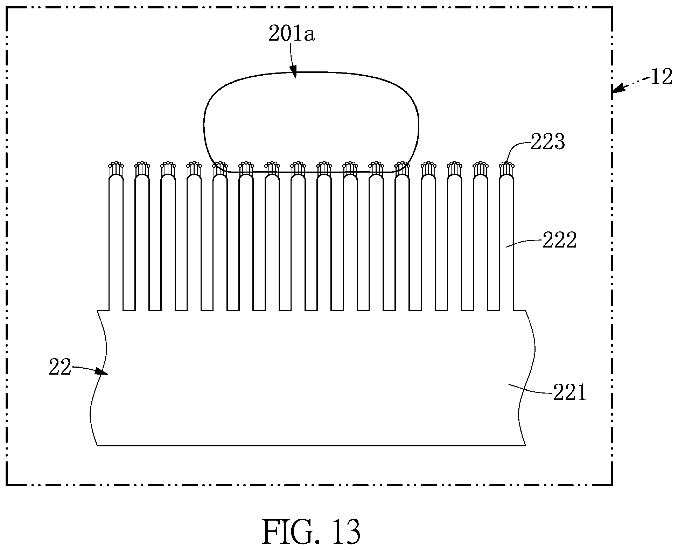

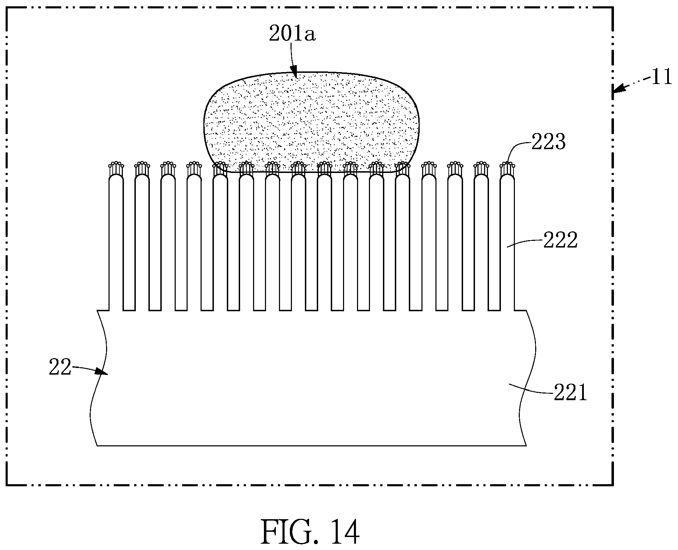

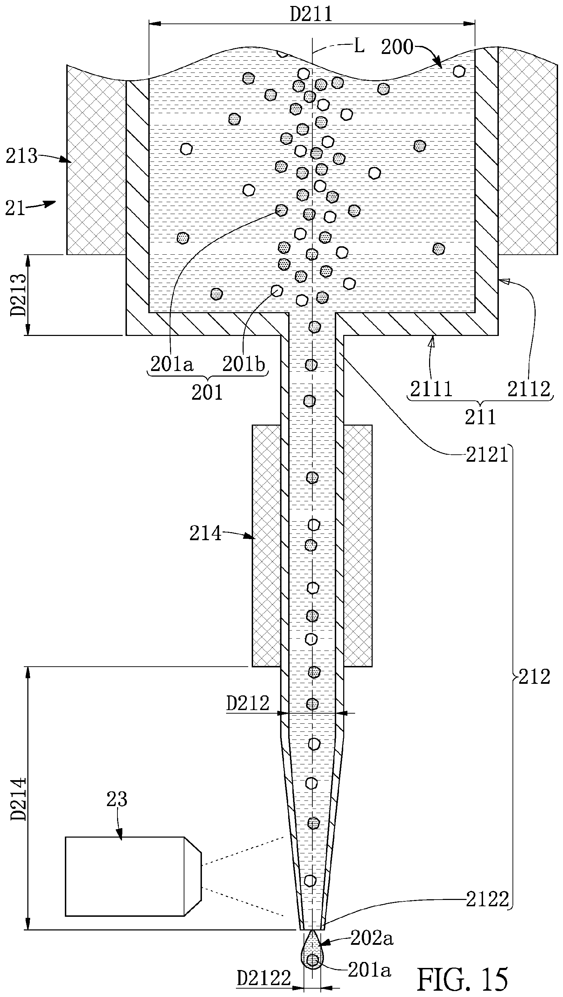

Referring to FIG. 1 to FIG. 15, a first embodiment of the present disclosure is provided. As shown in FIG. 1 and FIG. 2, the present embodiment provides a biological particle analysis method S100 and an identifying system 100. The identifying system 100 is applied to the biological particle analysis method S100, and the specific configuration of the identifying system 100 can be adjusted or changed according to design requirements, but the present disclosure is not limited thereto. For example, in other embodiments of the present disclosure not shown in the drawings, the biological particle analysis method S100 can be implemented through a structure other than the identifying system 100.

In the present embodiment, the identifying system 100 includes a multi-fluorescence staining apparatus 1, a pico-droplet generator 21, a camera device 23, and a biochip 22 that is provided to connect an operation of the multi-fluorescence staining apparatus 1 and an operation of the pico-droplet generator 21. The multi-fluorescence staining apparatus 1 includes a staining device 11, a washing device 12 arranged adjacent to the staining device 11, and a recording device 13 (e.g., a camera) that corresponds in position to the staining device 11, but the present disclosure is not limited thereto.

As shown in FIG. 1 and FIG. 3 to FIG. 14, the biological particle analysis method S 100 in the present embodiment sequentially includes a staining step S110, an analyzing step S120, a capturing step S130, a washing step S140, and a characterization expressing step S 150, thereby effectively completing an enrichment process and multiple biological characterization expressions for at least one target biological particle 201a that is selected from a liquid specimen 200 having a plurality of biological particles 201. In other words, any method not completely implementing the above steps in sequence is different from the biological particle analysis method S100 of the present embodiment.

As shown in FIG. 1, FIG. 3, and FIG. 4, the staining step S110 is implemented by fluorescence staining the liquid specimen 200 through a fluorescence staining process, so that at least one of the biological particles 201 becomes a fluorescence color and is defined as the at least one target biological particle 201a. In other words, the biological particles 201 are divided into the at least one target biological particle 201a and other non-target biological particles 201b.

In the present embodiment, the liquid specimen 200 is received in the specimen container 26, and the fluorescence staining process is implemented to the liquid specimen 200 in the specimen container 26 through the staining device 11 of the multi-fluorescence staining apparatus 1, but the present disclosure is not limited thereto. For example, in other embodiments of the present disclosure not shown in the drawings, the liquid specimen 200 in the specimen container 26 can be stained in other manners without using the staining device 11. Accordingly, the liquid specimen 200 being fluorescence stained is received in the specimen container 26.

Moreover, the liquid specimen 200 can be a body fluid from an animal (e.g., e.g., blood, lymph, saliva, ascites, or urine), and the at least one target biological particle 201a can be a specific type of cell, such as circulating tumor cells (CTCs), fetal nucleated red blood cells (FNRBCs), exosome, virus, or bacterium, but the present disclosure is not limited thereto. For example, in other embodiments of the present disclosure not shown in the drawings, the liquid specimen 200 can be chosen from plants.

As shown in FIG. 1 and FIG. 5 to FIG. 7, the analyzing step S 120 is implemented by accommodating the liquid specimen 200 being fluorescence stained into the pico-droplet generator 21, and using the camera device 23 to take a real-time image of the liquid specimen 200 in the pico-droplet generator 21. In the present embodiment, the pico-droplet generator 21 can be used to receive the liquid specimen 200 therein from the specimen container 26, and an immediate position of the at least one target biological particle 201a having the fluorescence color can be obtained by using the camera device 23 to take the real-time image.

As shown in FIG. 1 and FIG. 8 to FIG. 12, the capturing step S 130 is implemented by using the pico-droplet generator 21 to output a target pico-droplet 202a having the at least one target biological particle 201a onto the biochip 22 according to the real-time image. In other words, pico-droplets 202 generated from the liquid specimen 200 in the pico-droplet generator 21 in the present embodiment are divided into the target pico-droplet 202a and an abandoned pico-droplet 202b not having the target biological particle 201a. It should be noted that the pico-droplet 202, which has the non-target biological particle 201b but does not have the target biological particle 201a, is also defined as the abandoned pico-droplet 202b.

Specifically, whether the pico-droplet 202 outputted from the pico-droplet generator 21 has the at least one target biological particle 201a, which can be confirmed according to the real-time image, so that the pico-droplet 202 can be accurately placed onto the biochip 22 each time for enabling the at least one target biological particle 201a in the target pico-droplet 202a to be captured by the biochip 22, and the abandoned pico-droplet 202b is placed into an abandoned container 27, thereby effectively completing the enrichment process of the at least one target biological particle 201a.

It should be noted that the biochip 22 in the present embodiment is manufactured through a semi-conductor process, and the specific structure of the biochip 22 can be adjusted or changed according to design requirements. In order to clearly describe the biochip 22, the following description describes one kind of the biochip 22 having a better capturing effect, but the present disclosure is not limited thereto.

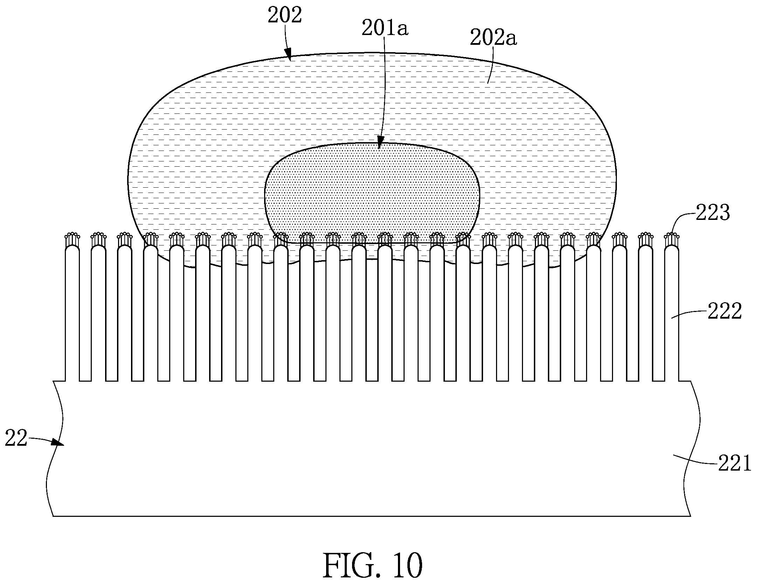

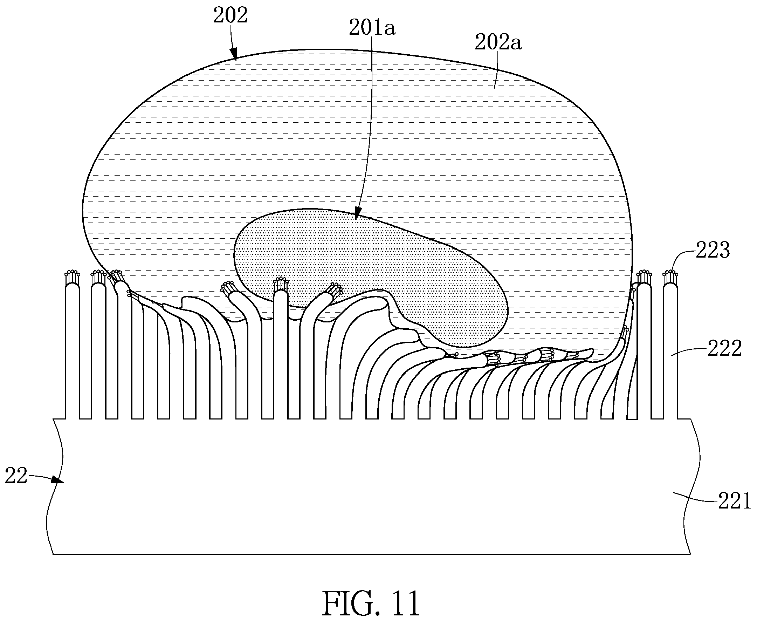

Specifically, as shown in FIG. 10 and FIG. 11, the biochip 22 is configured to carry the target pico-droplet 202a, and is capable of capturing the at least one target biological particle 201a in the target pico-droplet 202a. The biochip 22 includes a bottom layer 221, a plurality of capturing arms 222 connected to the bottom layer 221 and spaced apart from each other, and a surface modification layer 223 that is formed on ends of the capturing arms 222. In the capturing step S130, the biochip 22 is configured to capture the at least one target biological particle 201a through the capturing arms 222 and the surface modification layer 223.

Specifically, the surface modification layer 223 is preferably an arginylglycylaspartic acid (RGD) peptide layer, thereby facilitating capturing of the at least one target biological particle 201a in a chemical bonding manner, but the present disclosure is not limited thereto. Moreover, as shown in FIG. 11, at least two of the capturing arms 222 of the biochip 22 are elastically swingable with respect to the bottom layer 221 and are capable of clamping (or pinching) the at least one target biological particle 201a, thereby facilitating capturing of the at least one target biological particle 201a in a physical manner.

As shown in FIG. 1, FIG. 2, and FIG. 13, the washing step S140 is implemented by removing the fluorescent color of the at least one target biological particle 201a captured by the biochip 22 through a washing process. In the present embodiment, the washing process is implemented to the at least one target biological particle 201a captured by the biochip 22 through the washing device 12 of the multi-fluorescence staining apparatus 1.

As shown in FIG. 1, FIG. 2, and FIG. 14, the characterization expressing step S150 is implemented by fluorescence staining the at least one target biological particle 201a captured by the biochip 22 for N number of times through the fluorescence staining process and the washing process, and using the recording device 13 to obtain a plurality of fluorescence images respectively corresponding to N kinds of biological characterization expressions. In other words, each fluorescence staining of the at least one target biological particle 201a is related to (or corresponds to) one of the kinds of the biological characterization expressions. In the present embodiment, N is a positive integer within a range from 2 to 50, and N is preferably within a range from 4 to 28, but the present disclosure is not limited thereto.

In addition, the biochip 22 can be moved among different devices (e.g., the staining device 11, the washing device 12, and the carrying platform 25) through manpower or automation (e.g., by a robotic arm), but the present disclosure is not limited thereto.

In summary, the capturing step S130 of the biological particle analysis method S100 in the present embodiment can be implemented to achieve the enrichment effect by being cooperated with the staining step S110 and the analyzing step S 120. Moreover, the biological particle analysis method S 100 in the present embodiment can be implemented to effectively connect the capturing step S130, the washing step S140, and the characterization expressing step S150 by using the biochip 22 to firmly capture the at least one target biological particle 201a. Accordingly, the fluorescence images respectively corresponding to the N kinds of biological characterization expressions can be obtained from the liquid specimen 200 through the biological particle analysis method S100, thereby facilitating any evaluation and determination of the at least one target biological particle 201a. In addition, the biological particle analysis method S100 can be implemented such that the fluorescence images are overlapped with each other, thereby obtaining a biological characterization of the at least one target biological particle 201a.

As shown in FIG. 1, FIG. 2, and FIG. 15, the above description describes the steps S110-S150 of the biological particle analysis method S100 provided by the present embodiment, and in order to more clearly illustrate the present embodiment, a biological particle enrichment apparatus 2 that is cooperated with the multi-fluorescence staining apparatus 1 for jointly implementing the biological particle analysis method S 100 is further described, but the present disclosure is not limited thereto. For example, in other embodiments of the present disclosure not shown in the drawings, the biological particle enrichment apparatus 2 can be independently used (e.g., sold) or can be used in cooperation with other components.

In the present embodiment, the biological particle enrichment apparatus 2 includes the pico-droplet generator 21, the biochip 22 and the camera device 23 both corresponding in position to the pico-droplet generator 21, a controlling device 24 electrically coupled to the pico-droplet generator 21 and the camera device 23, a carrying platform 25 being capable of carrying the biochip 22 and corresponding in position to the pico-droplet generator 21, the specimen container 26 and the abandoned liquid container 27 both disposed on the carrying platform 25, and a pressure balance mechanism 28 that is connected to the pico-droplet generator 21, but the present disclosure is not limited thereto.

For example, in other embodiments of the present disclosure not shown in the drawings, types and a quantity of interior components of the biological particle enrichment apparatus 2 can be adjusted or changed according to design requirements, and the pico-droplet generator 21 can be independently used (e.g., sold) or can be used in cooperation with other components (e.g., the biochip 22).

The following description describes the structure and connection relationship of each component of the biological particle enrichment apparatus 2. In addition, part of the components (e.g., the biochip 22) of the biological particle enrichment apparatus 2 is described in the above description and will be omitted herein for the sake of brevity.

The pico-droplet generator 21 includes a container 211, a hollow needle 212 being in fluid communication with the container 211, a first piezoelectric member 213 disposed on the container 211, and a second piezoelectric member 214 that is disposed on the hollow needle 212. The container 211 is configured to receive the liquid specimen 200 and to transmit the liquid specimen 200 into the hollow needle 212, so that the hollow needle 212 can receive the liquid specimen 200 therein.

Specifically, the container 211 includes a bottom side 2111 and a surrounding lateral side 2112 that is connected to the bottom side 2111. The hollow needle 212 includes a connection end 2121 and a free end 2122 that is opposite to the connection end 2121. The connection end 2121 of the hollow needle 212 is (perpendicularly) connected to the bottom side 2111 of the container 211 so as to establish a fluid communication between the hollow needle 212 and the container 211. Moreover, an inner diameter D211 of the container 211 is within a range from 5 times to 30 times of an inner diameter D212 of the hollow needle 212. Specifically, the inner diameter D212 of the hollow needle 212 in the present embodiment that is measured is a portion other than the free end 2122 and is preferably within a range from 300 µm to 700 µm, and the free end 2122 of the hollow needle 212 in the present embodiment has an inner diameter D2122 being within a range from 30 µm to 150 µm, but the present disclosure is not limited thereto.

The first piezoelectric member 213 has a ring-shaped arrangement and is (annularly) disposed on the surrounding lateral side 2112 of the container 211, and the first piezoelectric member 213 in the present embodiment can be referred to as a top piezoelectric member 213. The first piezoelectric member 213 is configured to vibrate the container 211 for enabling the biological particles 201 in the container 211 to be moved along a direction away from the surrounding lateral side 2112. In other words, the first piezoelectric member 213 is configured to vibrate the container 211 for enabling the biological particles 201 in the container 211 to be arranged along a predetermined path.

Specifically, the predetermined path is preferably located along a central axis L of the hollow needle 212 (and a direction of gravity). Along a direction parallel to the central axis L, the first piezoelectric member 213 is spaced apart from the connection end 2121 by a distance D213 that is within a range from 0.2 cm to 2 cm. Moreover, the first piezoelectric member 213 is connected to and covers 20% to 85% of an area of the surrounding lateral side 2112 of the container 211. In addition, according to design requirements, the first piezoelectric member 213 can be a single one-piece structure having an annular shape or can be a structure having multiple components in an annular arrangement.

The second piezoelectric member 214 is disposed on an outer surface of the hollow needle 212, and the second piezoelectric member 214 in the present embodiment can be referred to as a bottom piezoelectric member 214. The second piezoelectric member 214 is configured to squeeze the hollow needle 212, so that the liquid specimen 200 flows outwardly and passes through the free end 2122 to form the pico-droplet 202. The pico-droplet 202 generated by the pico-droplet generator 21 can be further defined as a target pico-droplet 202a having the at least one target biological particle 201a or an abandoned pico-droplet 202b not having the target biological particle 201a.

Specifically, along a direction parallel to the central axis L, the second piezoelectric member 214 is spaced apart from the free end 2122 by a distance D214 that is within a range from 0.2 cm to 2 cm. The second piezoelectric member 214 is connected to and covers 20% to 85% of an area of the outer surface of the hollow needle 212. In addition, according to design requirements, the second piezoelectric member 214 can be a single one-piece structure having an annular shape or can be a structure having multiple components in an annular arrangement.

Furthermore, the pressure balance mechanism 28 is connected to the container 211, and the pressure balance mechanism 28 is configured to enable the liquid specimen 200 in the container 211 and the hollow needle 212 to be maintained at a predetermined pressure. The pressure balance mechanism 28 in the present embodiment is described as follows, but the present disclosure is not limited thereto. The pressure balance mechanism 28 includes an air pump 281, a switch 282 connected to the air pump 281, a pressure balance bottle 283 being in fluid communication with the air pump 281 and the switch 282, and a liquid injection bottle 284 that is in fluid communication with the switch 282 and the container 211.

The biochip 22, the specimen container 26, and the abandoned liquid container 27 are disposed on the carrying platform 25, and the carrying platform 25 and the pico-droplet generator 21 are movable relative to each other (e.g., the carrying platform 25 can be assembled with a multi-axis movable mechanism). Accordingly, the pico-droplet generator 21 (in the analyzing step S120) is moveable relative to the carrying platform 25 and is capable of sucking the liquid specimen 200 from the specimen container 26 through the free end 2122 of the hollow needle 212 (e.g., the liquid specimen 200 is sucked into the hollow needle 212 and the container 211). Moreover, the pico-droplet generator 21 is moveable relative to the carrying platform 25 so as to output the target pico-droplet 202a onto the biochip 22 and output the abandoned pico-droplet 202b into the abandoned liquid container 27.

The camera device 23 corresponds in position to the hollow needle 212, and the camera device 23 (in the analyzing step S120) is configured to take a real-time image of the liquid specimen 200 in the free end 2122. Moreover, the controlling device 24 is electrically coupled to the second piezoelectric member 214 and the camera device 23. According to the real-time image, the controlling device 24 (in the capturing step S130) is configured to drive the second piezoelectric member 214 when the at least one target biological particle 201a is located in the free end 2112, so that the second piezoelectric member 214 is driven to squeeze the hollow needle 212 to enable the liquid specimen 200 to flow outwardly and pass through the free end 2122 of the hollow needle 212 to form the target pico-droplet 202a.

In summary, the biological particle enrichment apparatus 2 of the present embodiment can be provided to enable the biological particles 201 to be moved toward a center of the container 211 through a vibration of the first piezoelectric member 213 (i.e., the top piezoelectric member 213), thereby preventing the biological particles 201 from being adhered to inner walls of the container 211 and completing the enrichment process of the target biological particle 201a.

Specifically, the biological particle analysis method S100 or the biological particle enrichment apparatus 2 in the present embodiment is provided with the cooperation between the camera device 23 and the second piezoelectric member 214 (i.e., the bottom piezoelectric member 214), so that according to the real-time image of the liquid specimen 200 in the free end 2122, the second piezoelectric member 214 can be driven to form the target pico-droplet 202a when the at least one target biological particle 201a is located in the free end 2122, thereby effectively completing the enrichment process of the target biological particle 201a.

### [Second Embodiment]

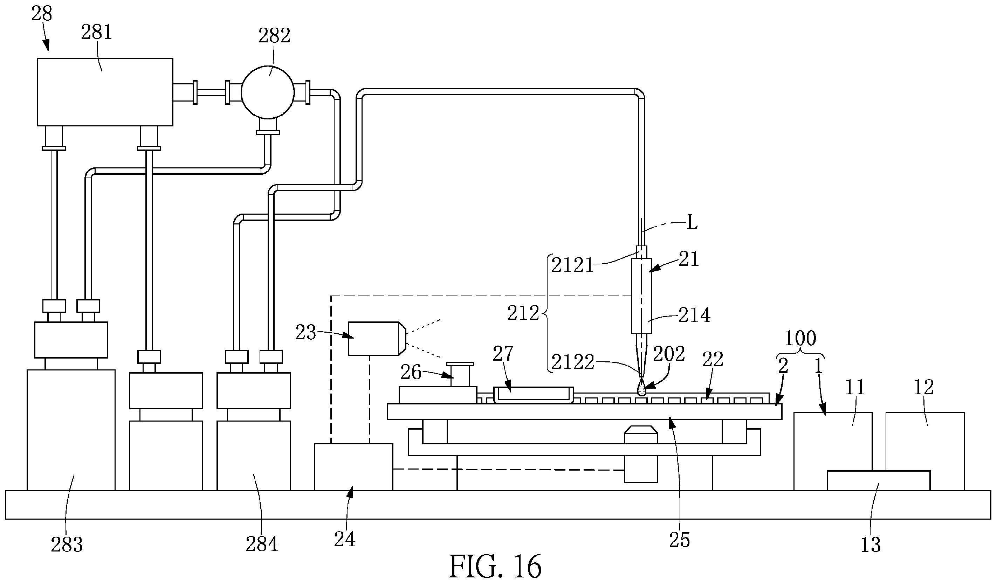

Referring to FIG. 16, a second embodiment of the present disclosure, which is similar to the first embodiment of the present disclosure, is provided. For the sake of brevity, descriptions of the same components in the first and second embodiments of the present disclosure will be omitted herein, and the following description only discloses different features between the first and second embodiments.

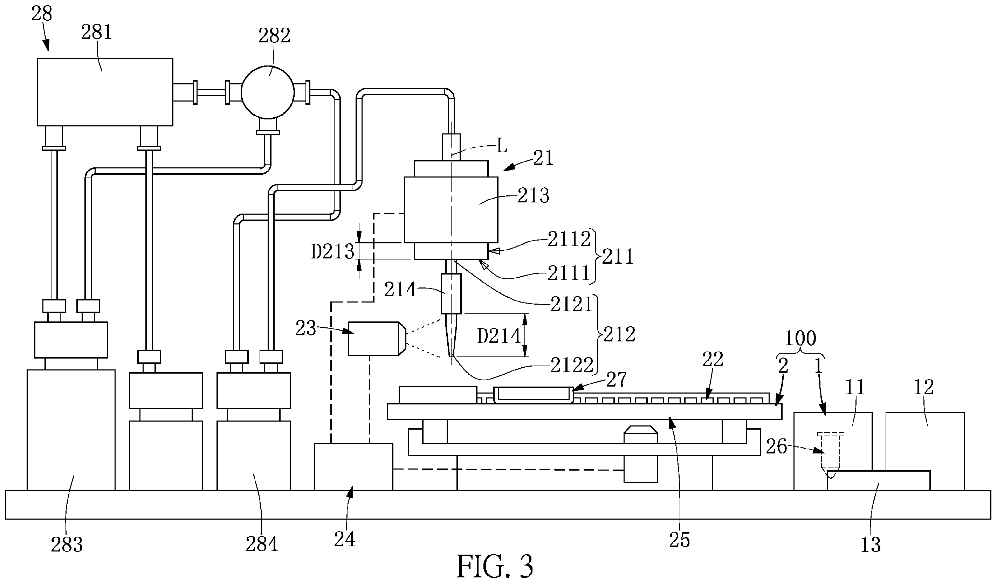

In the present embodiment, the pico-droplet generator 21 has a single piezoelectric member. In other words, the pico-droplet generator 21 in the present embodiment is provided without the first piezoelectric member 213 (i.e., the top piezoelectric member 213) and the container 211 shown in FIG. 3 of the first embodiment. Accordingly, the pico-droplet generator 21 in the present embodiment includes the hollow needle 212 and the second piezoelectric member 214 (i.e., the bottom piezoelectric member 214) that is disposed on the outer surface of the hollow needle 212.

Specifically, the connection end 2121 of the hollow needle 212 is connected to the pressure balance mechanism 28 (e.g., the liquid injection bottle 284), and the hollow needle 212 receives the liquid specimen 200 therein. Accordingly, in the capturing step S130, the second piezoelectric member 214 (i.e., the bottom piezoelectric member 214) of the pico-droplet generator 21 is configured to squeeze the hollow needle 212, so that the liquid specimen 200 flows outwardly and passes through the free end 2122 to form the target pico-droplet 202a, but the present disclosure is not limited thereto. For example, in other embodiments of the present disclosure not shown in the drawings, the pico-droplet generator 21 can be a structure operated in a manner other than the piezoelectric manner according to design requirements.

### [Beneficial Effects of the Embodiments]

In conclusion, the capturing step of the biological particle analysis method in the present disclosure can be implemented to achieve the enrichment effect by being cooperated with the staining step and the analyzing step. Moreover, the biological particle analysis method in the present disclosure can be implemented to effectively connect the capturing step, the washing step, and the characterization expressing step by using the biochip to capture the at least one target biological particle. Accordingly, the fluorescence images respectively corresponding to multiple kinds of biological characterization expressions can be obtained from the liquid specimen through the biological particle analysis method, thereby facilitating any evaluation and determination of the at least one target biological particle.

In addition, the biological particle enrichment apparatus of the present disclosure can be provided to enable the biological particles to be moved toward a center of the container through a vibration of the first piezoelectric member (i.e., the top piezoelectric member), thereby preventing the biological particles from being adhered to inner walls of the container and facilitating completing the enrichment process of the target biological particle.

Specifically, the biological particle analysis method or the biological particle enrichment apparatus in the present disclosure is provided with the cooperation between the camera device and the second piezoelectric member (i.e., the bottom piezoelectric member), so that according to the real-time image of the liquid specimen in the free end, the second piezoelectric member can be driven to form the target pico-droplet when the at least one target biological particle is located in the free end, thereby effectively completing the enrichment process of the target biological particle.

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present disclosure pertains without departing from its scope.

## Claims

1. A biological particle analysis method, comprising:
a staining step (S110) implemented by fluorescence staining a liquid specimen (200) having a plurality of biological particles (201) through a fluorescence staining process, so that at least one of the biological particles (201) becomes a fluorescence color and is defined as at least one target biological particle (201a);
an analyzing step (S120) implemented by accommodating the liquid specimen (200) being fluorescence stained into a pico-droplet generator (21), and using a camera device (23) to take a real-time image of the liquid specimen (200) in the pico-droplet generator (21), wherein the pico-droplet generator (21) includes a hollow needle for receiving the liquid specimen therein, a container (211) and a top piezoelectric member (213) disposed on the container (211), the container (211) receives the liquid specimen (200), and the hollow needle (212) is in fluid communication with the container by being connected to a bottom side (2111) of the container (211), wherein the top piezoelectric member (213) is configured to vibrate the container (211) for enabling the biological particles (201) in the container (211) to be arranged along a predetermined path of the hollow needle (212), and wherein the camera device (23) corresponds in position to the hollow needle (212), and the camera device (23) in the analyzing step (S120) is used to take the real-time image of the liquid specimen (200) in a free end (2122) of the hollow needle (212);
a capturing step (S130) implemented by using the pico-droplet generator (21) to output a target pico-droplet (202a) having the at least one target biological particle (201a) onto a biochip (22) according to the real-time image, wherein the at least one target biological particle (201a) in the target pico-droplet (202a) is captured by the biochip (22);
a washing step (S140) implemented by removing the fluorescent color of the at least one target biological particle (201a) in the target pico-droplet (202a) captured by the biochip (22) through a washing process; and
a characterization expressing step (S150) implemented by fluorescence staining the at least one target biological particle (201a) captured by the biochip (22) for N number of times through the fluorescence staining process and the washing process, and using a recording device (13) to obtain a plurality of fluorescence images respectively corresponding to N kinds of biological characterization expressions, wherein N is a positive integer within a range from 2 to 50 and wherein each fluorescence staining of the at least one target biological particle is related to one of the N kinds of biological characterization expressions.

2. The biological particle analysis method according to claim 1, wherein the pico-droplet generator (21) includes a bottom piezoelectric member (214) disposed on an outer surface of the hollow needle (212), and the hollow needle (212) receives the liquid specimen (200) therein, and wherein, in the capturing step (S130), the bottom piezoelectric member (214) of the pico-droplet generator (21) is operated to squeeze the hollow needle (212), so that the liquid specimen (200) flows outwardly and passes through the free end (2122) of the hollow needle (212) to form the target pico-droplet (202a).

3. The biological particle analysis method according to claim 1, wherein, in the capturing step (S130), according to the real-time image, a controlling device (24) electrically coupled to the bottom piezoelectric member (214) and the camera device (23) is configured to drive the bottom piezoelectric member (214) when the at least one target biological particle (201a) is located in the free end (2122).

4. The biological particle analysis method according to claim 2, wherein, in the staining step (S110), the liquid specimen (200) being fluorescence stained is received in a specimen container (26) disposed on a carrying platform (25); and in the analyzing step (S120), the pico-droplet generator (21) is movable relative to the carrying platform (25) and is capable of sucking the liquid specimen (200) from the specimen container (26) through the free end (2122).

5. The biological particle analysis method according to claim 4, wherein the biochip (22) and an abandoned liquid container (27) are disposed on the carrying platform (25), and the pico-droplet generator (21) is moveable relative to the carrying platform (25) so as to output the target pico-droplet (202a) onto the biochip (22) and output an abandoned pico-droplet (202b) not having the target biological particle (201a) into the abandoned liquid container (27).

6. The biological particle analysis method according to claim 1, wherein the biochip (22) includes a bottom layer (221), a plurality of capturing arms (222) connected to the bottom layer (221) and spaced apart from each other, and a surface modification layer (223) that is formed on ends of the capturing arms (222), and wherein, in the capturing step (S130), the biochip (22) is configured to capture the at least one target biological particle (201a) through the capturing arms (222) and the surface modification layer (223).

7. The biological particle analysis method according to claim 6, wherein the surface modification layer (223) is an arginylglycylaspartic acid (RGD) peptide layer.

8. The biological particle analysis method according to claim 6, wherein, in the capturing step (S130), at least two of the capturing arms (222) of the biochip (22) are elastically swingable with respect to the bottom layer (221) and are capable of clamping the at least one target biological particle (201a).

## Patentansprüche

1. Verfahren zur biologischen Partikelanalyse, umfassend:
einen Färbeschritt (S110), der durch Fluoreszenzfärbung einer flüssigen Probe (200) mit einer Vielzahl von biologischen Partikeln (201) durch einen Fluoreszenzfärbeprozess durchgeführt wird, so dass mindestens eines der biologischen Partikel (201) eine Fluoreszenzfarbe annimmt und als mindestens ein biologisches Target-Partikel (201a) definiert wird;
einen Analyseschritt (S120), der durchgeführt wird, indem die flüssige Probe (200), die fluoreszenzgefärbt wird, in einem Pikotropfen-Generator (21) untergebracht wird, und eine Kameravorrichtung (23) verwendet wird, um ein Echtzeitbild der flüssigen Probe (200) in dem Pikotropfen-Generator (21) aufzunehmen, wobei der Pikotropfen-Generator (21) Folgendes umfasst
eine Hohlnadel (212) zur Aufnahme der flüssigen Probe (200),
einen Behälter (211) und ein oberes piezoelektrisches Element (213), das auf dem Behälter (211) angeordnet ist,
wobei der Behälter (211) die flüssige Probe (200) aufnimmt und die Hohlnadel (212) in Fluidverbindung mit dem Behälter steht, indem sie mit einer Unterseite (2111) des Behälters (211) verbunden ist,
wobei das obere piezoelektrische Element (213) so konfiguriert ist, dass es den Behälter (211) in Schwingung versetzt, um zu ermöglichen, dass die biologischen Partikel (201) in dem Behälter (211) entlang eines vorbestimmten Weges der Hohlnadel (212) angeordnet werden, und
wobei die Kameravorrichtung (23) in ihrer Position zur Hohlnadel (212) korrespondiert und die Kameravorrichtung (23) in dem Analyseschritt (S120) verwendet wird, um das Echtzeitbild der flüssigen Probe (200) in einem freien Ende (2122) der Hohlnadel (212) aufzunehmen;
einen Einfangschritt (S130), der unter Verwendung des Pikotropfen-Generators (21) implementiert wird, um ein Target-Pikotropfen (202a) mit dem mindestens einen biologischen Target-Partikel (201a) auf einen Biochip (22) gemäß dem Echtzeitbild auszugeben, wobei der mindestens eine biologische Target-Partikel (201a) in dem Target-Pikotropfen (202a) von dem Biochip (22) eingefangen wird;
einen Waschschritt (S140), der durch Entfernen der fluoreszierenden Farbe des mindestens einen biologischen Target-Teilchens (201a) in dem von dem Biochip (22) eingefangenen Target-Pikotropfen (202a) durch einen Waschprozess durchgeführt wird; und
einen Charakterisierungs-Expressionsschritt (S150), durchgeführt durch Fluoreszenzfärbung des mindestens einen biologischen Target-Partikels 201a, das durch den Biochip (22) eingefangen wurde, für eine Anzahl von N Malen durch den Fluoreszenzfärbeprozess und den Waschprozess und unter Verwendung der Aufzeichnungsvorrichtung (13), um eine Vielzahl von Fluoreszenzbildern zu erhalten, die jeweils N Arten von biologischen Charakterisierungs-Expressionen entsprechen,
wobei N eine positive ganze Zahl in einem Bereich von 2 bis 50 und,
wobei jede Fluoreszenzfärbung des mindestens einen biologischen Target-Partikels in Beziehung zu einer der N Arten von biologischen Charakterisierungs-Expressionen steht.

2. Verfahren zur biologischen Partikelanalyse nach Anspruch 1, wobei der Pikotropfen-Generator (21) ein unteres piezoelektrisches Element (214) umfasst, das an einer Außenfläche der Hohlnadel (212) angeordnet ist, und die Hohlnadel (212) die flüssige Probe (200) darin aufnimmt, und wobei in dem Einfangschritt (S130) das untere piezoelektrische Element (214) des Pikotropfen-Generators (21) betätigt wird, um die Hohlnadel (212) zusammenzudrücken, so dass die flüssige Probe (200) nach außen fließt und durch das freie Ende (2122) der Hohlnadel (212) hindurchgeht, um den Target-Pikotropfen (202a) zu bilden.

3. Verfahren zur biologischen Partikelanalyse nach Anspruch 1, wobei in dem Einfangschritt (S130) eine Steuervorrichtung (24), die elektrisch mit dem unteren piezoelektrischen Element (214) und der Kameravorrichtung (23) gekoppelt ist, so konfiguriert ist, dass sie das untere piezoelektrische Element (214) ansteuert, wenn sich das mindestens eine biologische Target-Partikel (201a) gemäß dem Echtzeitbild in dem freien Ende (2122) befindet.

4. Verfahren zur biologischen Partikelanalyse nach Anspruch 2, wobei in dem Färbeschritt (S110) die flüssige Probe (200), die fluoreszenzgefärbt ist, in einem Probenbehälter (26) aufgenommen wird, der auf einer Trageplattform (25) angeordnet ist; und in dem Analyseschritt (S120) der Pikotropfen-Generator (21) relativ zu der Trageplattform (25) beweglich ist und in der Lage ist, die flüssige Probe (200) aus dem Probenbehälter (26) durch das freie Ende (2122) anzusaugen.

5. Verfahren zur biologischen Partikelanalyse nach Anspruch 4, wobei der Biochip (22) und ein Flüssigabfallbehälter (27) auf der Trageplattform (25) angeordnet sind, und der Pikotropfen-Generator (21) relativ zu der Trageplattform (25) beweglich ist, um den Target-Pikotropfen (202a) auf den Biochip (22) auszugeben und einen zu verwerfenden Pikotropfen (202b), der nicht das biologische Target-Partikel (201a) enthält, in den Flüssigabfallbehälter (27) auszugeben.

6. Verfahren zur biologischen Partikelanalyse nach Anspruch 1, wobei der Biochip (22) eine untere Schicht (221), eine Vielzahl von Einfangarmen (222), die mit der unteren Schicht (221) verbunden und voneinander beabstandet sind, und eine Oberflächenmodifikationsschicht (223), die an den Enden der Einfangarme (222) ausgebildet ist, umfasst, und wobei der Biochip (22) in dem Einfangschritt (S130) so konfiguriert ist, dass er das mindestens eine biologische Target-Partikel (201a) durch die Einfangarme (222) und die Oberflächenmodifikationsschicht (223) einfängt.

7. Verfahren zur biologischen Partikelanalyse nach Anspruch 6, wobei die Oberflächenmodifikationsschicht (223) eine Arginylglycylasparaginsäure (RGD)-Peptidschicht ist.

8. Verfahren zur biologischen Partikelanalyse nach Anspruch 6, wobei in der Schritt des Einfangens (S130) mindestens zwei der Einfangarme (222) des Biochips (22) in Bezug auf die untere Schicht (221) elastisch schwenkbar und in der Lage sind, das mindestens eine biologische Target-Partikel (201a) einzuklemmen.

## Revendications

1. Procédé d'analyse de particules biologiques, comprenant :
une étape de coloration (S110) mise en œuvre par coloration par fluorescence d'un échantillon liquide (200) contenant une pluralité de particules biologiques (201) au moyen d'un processus de coloration par fluorescence, de sorte qu'au moins une des particules biologiques (201) prenne une couleur fluorescente et soit définie comme au moins une particule biologique cible (201a) ;
une étape d'analyse (S120) mise en œuvre en plaçant l'échantillon liquide (200) coloré par fluorescence dans un générateur de pico-gouttelettes (21) et en utilisant un dispositif de caméra (23) pour prendre une image en temps réel de l'échantillon liquide (200) dans le générateur de pico-gouttelettes (21), dans lequel le générateur de pico-gouttelettes (21) comprend une aiguille creuse pour recevoir l'échantillon liquide à l'intérieur,
un récipient (211) et un élément piézoélectrique supérieur (213) disposé sur le récipient (211), le récipient (211) recevant l'échantillon liquide (200), et l'aiguille creuse (212) étant en communication fluidique avec le récipient en étant reliée à un côté inférieur (2111) du récipient (211), dans lequel l'élément piézoélectrique supérieur (213) est configuré pour faire vibrer le récipient (211) afin de permettre aux particules biologiques (201) dans le récipient (211) d'être disposées le long d'un trajet prédéterminé de l'aiguille creuse (212), et dans lequel le dispositif de caméra (23) correspond en position à l'aiguille creuse (212), et le dispositif de caméra (23) dans l'étape d'analyse (S120) est utilisé pour prendre l'image en temps réel de l'échantillon liquide (200) dans une extrémité libre (2122) de l'aiguille creuse (212) ;
une étape de capture (S130) mise en œuvre en utilisant le générateur de pico-gouttelettes (21) pour émettre une pico-gouttelette cible (202a) comportant ladite au moins une particule biologique cible (201a) sur une biopuce (22) en fonction de l'image en temps réel, où ladite au moins une particule biologique cible (201a) dans la pico-gouttelette cible (202a) est capturée par la biopuce (22) ;
une étape de lavage (S140) mise en œuvre en éliminant la couleur fluorescente de ladite au moins une particule biologique cible (201a) dans la pico-gouttelette cible (202a) capturée par la biopuce (22) par un processus de lavage ; et
une étape d'expression de caractérisation (S150) mise en œuvre en colorant par fluorescence ladite au moins une particule biologique cible (201a) capturée par la biopuce (22) N fois par le processus de coloration par fluorescence et le processus de lavage, et en utilisant un dispositif d'enregistrement (13) pour obtenir une pluralité d'images de fluorescence correspondant respectivement à N types d'expressions de caractérisation biologique, où N est un nombre entier positif compris dans une plage de 2 à 50 et où chaque coloration par fluorescence de ladite au moins une particule biologique cible est liée à l'un des N types d'expressions de caractérisation biologique.

2. Procédé d'analyse de particules biologiques selon la revendication 1, dans lequel le générateur de pico-gouttelettes (21) comprend un élément piézoélectrique inférieur (214) disposé sur une surface extérieure de l'aiguille creuse (212), et l'aiguille creuse (212) reçoit l'échantillon liquide (200) à l'intérieur, et dans lequel, dans l'étape de capture (S130), l'élément piézoélectrique inférieur (214) du générateur de pico-gouttelettes (21) est actionné pour comprimer l'aiguille creuse (212), de sorte que l'échantillon liquide (200) s'écoule vers l'extérieur et passe à travers l'extrémité libre (2122) de l'aiguille creuse (212) pour former la pico-gouttelette cible (202a).

3. Procédé d'analyse de particules biologiques selon la revendication 1, dans lequel, dans l'étape de capture (S130), en fonction de l'image en temps réel, un dispositif de commande (24) couplé électriquement à l'élément piézoélectrique inférieur (214) et au dispositif de caméra (23) est configuré pour entraîner l'élément piézoélectrique inférieur (214) lorsque ladite au moins une particule biologique cible (201a) est située dans l'extrémité libre (2122).

4. Procédé d'analyse de particules biologiques selon la revendication 2, dans lequel, dans l'étape de coloration (S110), l'échantillon liquide (200) soumis à une coloration par fluorescence est reçu dans un récipient à échantillon (26) disposé sur une plate-forme de transport (25) ; et dans l'étape d'analyse (S120), le générateur de pico-gouttelettes (21) est mobile par rapport à la plate-forme de transport (25) et est capable d'aspirer l'échantillon liquide (200) du récipient d'échantillon (26) à travers l'extrémité libre (2122).

5. Procédé d'analyse de particules biologiques selon la revendication 4, dans lequel la biopuce (22) et un récipient de liquide abandonné (27) sont disposés sur la plate-forme de transport (25), et le générateur de pico-gouttelettes (21) est mobile par rapport à la plate-forme de transport (25) de manière à émettre la pico-gouttelette cible (202a) sur la biopuce (22) et à émettre une pico-gouttelette abandonnée (202b) ne contenant pas la particule biologique cible (201a) dans le récipient de liquide abandonné (27).

6. Procédé d'analyse de particules biologiques selon la revendication 1, dans lequel la biopuce (22) comprend une couche inférieure (221), une pluralité de bras de capture (222) reliés à la couche inférieure (221) et espacés les uns des autres, et une couche de modification de surface (223) qui est formée sur des extrémités des bras de capture (222), et dans lequel, dans l'étape de capture (S130), la biopuce (22) est configurée pour capturer ladite au moins une particule biologique cible (201a) à travers les bras de capture (222) et la couche de modification de surface (223).

7. Procédé d'analyse de particules biologiques selon la revendication 6, dans lequel la couche de modification de surface (223) est une couche de peptide d'acide arginylglycylaspartique (RGD).

8. Procédé d'analyse de particules biologiques selon la revendication 6, dans lequel, dans l'étape de capture (S130), au moins deux des bras de capture (222) de la biopuce (22) peuvent osciller élastiquement par rapport à la couche inférieure (221) et sont capables de serrer ladite au moins une particule biologique cible (201a).
